(19) Europäisches Patentamt

European Patent Office

Office européen des brevets

(11) EP 0 578 585 B1

## (12) FASCICULE DE BREVET EUROPEEN

(45) Date de publication et mention de la délivrance du brevet:
**16.10.1996  Bulletin 1996/42**

(51) Int. Cl.⁶: **A61M 1/16**

(21) Numéro de dépôt: **93420264.9**

(22) Date de dépôt: **22.06.1993**

(54) **Procédé de vérification du fonctionnement de capteurs situés sur un circuit de liquide de dialyse**

Verfahren zur Funktionsprüfung von Sensoren an einem Dialyse Flüssigkeitskreislauf

Process for checking the operation of sensors on a dialysis fluid circuit

(84) Etats contractants désignés:
**BE DE ES FR GB IT NL**

(30) Priorité: **06.07.1992 FR 9208564**

(43) Date de publication de la demande:
**12.01.1994  Bulletin 1994/02**

(73) Titulaire: **HOSPAL INDUSTRIE**
**F-69330 Meyzieu (FR)**

(72) Inventeurs:
• **Goux, Nicolas**
  **F-69290 Craponne (FR)**
• **Bene, Bernard**
  **F-69540 Irigny (FR)**

(56) Documents cités:
**EP-A- 0 097 366**      **EP-A- 0 291 421**
**EP-A- 0 428 927**      **US-A- 5 110 477**

## Description

La présente invention concerne le domaine technique du traitement extracorporel du sang par dialyse. Plus particulièrement la présente invention a pour objet un procédé de vérification du fonctionnement de capteurs situés sur le circuit de liquide de dialyse d'un rein artificiel.

En effet, il existe sur le circuit de liquide de dialyse d'un rein artificiel de nombreux capteurs permettant de mesurer ou de vérifier un certain nombre de caractéristiques du liquide de dialyse, telles que, par exemple, la conductivité ou la concentration en sels. De telles caractéristiques sont intéressantes à connaitre et à suivre au cours d'une séance de traitement, car d'une part l'efficacité de l'épuration du sang est fonction de la composition du liquide de dialyse utilisé et d'autre part, il est possible dans certains cas de déduire la valeur de certaines caractéristiques du sang grâce à des mesures effectuées sur le liquide de dialyse.

On peut notamment, à partir de mesures de la conductivité du liquide de dialyse, déterminer la concentration en sodium du sang circulant de l'autre côté de la membrane, ce qui évite de prélever au patient des échantillons de sang. La mesure de la conductivité peut être effectuée sur du liquide de dialyse recirculant dans une portion du circuit jusqu'à obtention d'un équilibre avec le sang, ainsi que cela est décrit dans l'article "Optimization of Sodium Concentration by Plasma Water Conductivity Monitoring" de Petitclerc, Man, Goureau, Jehenne et Funck-Brentano (Progress in Artificial Organs - 1985), ou, de façon plus intéressante, grâce à deux mesures effectuées respectivement en amont et en aval du dispositif utilisé pour le traitement du sang, les deux mesures pouvant éventuellement être réalisées grâce à un seul conductimètre baigné alternativement par le liquide de dialyse frais et par le liquide de dialyse usagé. Les déterminations de natrémie ainsi effectuées peuvent ensuite être utilisées pour déterminer la composition de liquide de dialyse la plus favorable en fonction de l'épuration souhaitée. Il est donc très important que les informations fournies par le conductimètre soient des informations fiables. Or, les moyens habituels de vérification du fonctionnement des conductimètres imposent un temps important d'immobilisation du rein artificiel, ce qui diminue les possibilités de traitement de patients.

Le but de la présente invention est de pallier cet inconvénient et de proposer un procédé permettant de vérifier le fonctionnement de capteurs présents sur le circuit de liquide de dialyse sans entrainer d'immobilisation particulière de l'appareil de dialyse.

Pour atteindre ce but, on prévoit, selon l'invention, un procédé de vérification du fonctionnement d'au moins un capteur situé sur un circuit de liquide de dialyse d'un rein artificiel comportant un échangeur à deux compartiments séparés par une membrane semi-perméable, un des compartiments étant relié au circuit de liquide de dialyse alors que l'autre compartiment est destiné à être relié au circuit extracorporel de sang, ce procédé étant caractérisé en ce qu'il consiste à

- faire circuler dans le circuit de liquide de dialyse un liquide de dialyse,
- faire circuler dans le circuit extracorporel de sang un liquide témoin de caractéristiques connues,
- effectuer au moins une mesure grâce à au moins un capteur d'au moins une caractéristique du liquide de dialyse,
- calculer à partir de cette mesure, la valeur théorique d'au moins une caractéristique du liquide témoin,
- comparer la valeur théorique calculée à la valeur connue de la caractéristique du liquide témoin,
- conclure au bon fonctionnement du capteur au moyen duquel la mesure a été effectuée lorsque la valeur calculée de la caractéristique du liquide témoin est sensiblement égale à la valeur connue de caractéristique du liquide témoin.

Selon une caractéristique particulière de l'invention, le liquide témoin est une solution stérile de sérum physiologique. Ainsi, les caractéristiques de cette solution sont parfaitement connues et peuvent être utilisées pour vérifier la justesse des moyens de mesure situés sur le circuit de liquide de dialyse.

Selon une autre caractéristique de l'invention, le procédé de vérification est mis en oeuvre lors de la phase de rinçage et d'amorçage de l'hémodialyseur.

Il est donc possible, sans introduire d'étape supplémentaire, de vérifier le bon fonctionnement des capteurs au début de chaque séance de traitement.

D'autres particularités et avantages de la présente invention apparaitront à la lecture de la description qui va suivre en référence aux schémas sur lesquels ne sont représentés que les éléments nécessaires à la compréhension de la présente invention.

La figure 1 illustre un premier mode de réalisation de la présente invention.

La figure 2 illustre un second mode de réalisation de la présente invention.

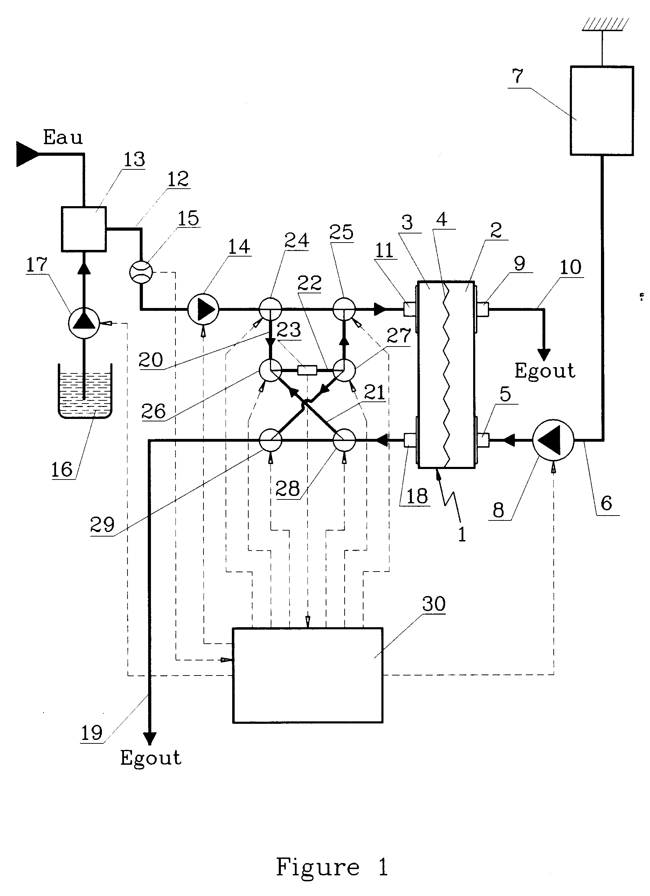

Le rein artificiel représenté sur la figure 1 comporte un hémodialyseur 1 ayant deux compartiments 2,3 séparés par une membrane semi-perméable 4 permettant la dialyse du sang. Le compartiment 2 est destiné à la circulation du sang à traiter alors que le compartiment 3 est destiné à la circulation du liquide de dialyse.

Durant l'opération de vérification illustrée sur cette figure, l'entrée 5 du compartiment 2 est reliée par une canalisation 6 à une source 7 de liquide témoin dont la composition est parfaitement déterminée. Une pompe 8 assure la circulation du liquide témoin. La sortie 9 du compartiment 2 est reliée par une canalisation 10 à l'égout.

Le compartiment 3 de l'hémodialyseur possède une entrée 11 reliée par une canalisation d'alimentation 12 à une source 13 de liquide de dialyse. La canalisation 12 comporte un débitmètre 15 et une pompe 14 qui assure la circulation du liquide de dialyse.

La source 13 de liquide de dialyse est alimentée en eau et en une solution concentrée présente dans un réservoir 16. Le débit d'alimentation de la source 13 en solution concentrée est réglé par une pompe 17. La sortie 18 du compartiment 3 est reliée à une canalisation 19 d'évacuation du liquide de dialyse usagé à l'égout.

La canalisation d'alimentation 12 et la canalisation d'évacuation 19 possèdent chacune un circuit de dérivation, respectivement 20 et 21. Ces deux circuits de dérivation comportent une portion commune 22 équipée d'un capteur 23 constitué par exemple par une électrode spécifique au sodium.

Un ensemble approprié de vannes 24, 25, 26, 27, 28, 29 permet de relier sélectivement la portion commune 22 équipée du capteur 23 à la canalisation 12 d'alimentation en liquide de dialyse frais ou à la canalisation 19 d'évacuation du liquide de dialyse usagé.

Une unité de contrôle 30 reçoit des informations en provenance du débitmètre 15 et du capteur 23 ; cette unité commande la positionnement des vannes 24, 25, 26, 27, 28 et 29, ainsi que le fonctionnement des pompes 8, 14 et 17.

Préalablement à la mise en oeuvre du rein artificiel ainsi décrit, l'opérateur fournit à l'unité 30 des informations concernant les caractéristiques de l'hémodialyseur utilisé ainsi que des consignes relatives au débit de la pompe 8, au débit de la pompe 14 et à la conductivité souhaitée du liquide de dialyse.

Ensuite, le fonctionnement du rein artificiel est le suivant. Le liquide témoin 7 est mis en circulation grâce à l'action de la pompe 8 ce liquide pénètre dans l'hémodialyseur par l'entrée 5 pour en ressortir par la sortie 9 avant d'être évacué à l'égout. Le liquide témoin utilisé peut avantageusement être la solution stérile de sérum physiologique employée pour rincer et remplir le circuit de circulation extracorporelle du sang à traiter.

Pendant ce temps, l'unité 30 commande le fonctionnement de la pompe 17 en fonction de la consigne de conductivité établie par l'opérateur. Le concentré est dilué grâce à de l'eau alimentant la source 13. Le liquide de dialyse ainsi préparé est, après chauffage grâce à un dispositif approprié (non représenté) mis en circulation grâce à la pompe 14. Le fonctionnement de cette pompe est commandé par l'unité 30 en réponse notamment aux valeurs de débit qui lui sont transmises par le débitmètre 15. Le liquide de dialyse pénètre dans le compartiment 3 de l'hémodialyseur par l'entrée 11 puis en ressort par la sortie 18 avant d'être conduit à l'égout par l'intermédiaire de la canalisation 19. A l'intérieur de l'hémodialyseur, des échanges ont lieu entre les deux liquides par dialyse au travers de la membrane 4, le liquide de forte concentration en une certaine substance ayant tendance à s'appauvrir au profit du liquide de plus faible concentration.

La quantité de substance transférée au travers de la membrane est représentée par la dialysance qui est une caractéristique propre à chaque membrane.

La formule d'obtention de la dialysance est la suivante :

$$D = \frac{Qd\,(C_{ind} - C_{outd})}{C_{ind} - C_{inb}} \qquad (1)$$

Dans cette formule :

Qd     est le débit de liquide de dialyse mesuré par le débitmètre 15

$C_{ind}$     est la concentration en une substance à l'entrée du compartiment 3 de l'hémodialyseur

$C_{outd}$     est la concentration en la même substance à la sortie du compartiment 3 de l'hémodialyseur

$C_{inb}$     est la concentration en la même substance à l'entrée du compartiment 2 de l'hémodialyseur.

Lorsque l'on fait varier la concentration du liquide de dialyse à l'entrée de $C_{ind1}$ à $C_{ind2}$, on obtient une variation de la concentration à la sortie qui passe de $C_{outd1}$ à $C_{outd2}$.

L'application de la formule d'obtention de la dialysance à ces deux concentrations conduit à l'égalité suivante :

$$D = \frac{Qd\,(C_{ind1} - C_{outd1})}{C_{ind1} - C_{inb}} = \frac{Qd\,(C_{ind2} - C_{outd2})}{C_{ind2} - C_{inb}} \quad (2)$$

Le débit de liquide de dialyse Qd étant maintenu constant, on peut en déduire la concentration du liquide circulant dans le compartiment 2 de l'hémodialyseur.

$$C_{inb} = \frac{(C_{outd1} \cdot C_{ind2}) - (C_{outd2} \cdot C_{ind1})}{(C_{outd1} - C_{ind1}) + (C_{ind2} - C_{outd2})} \quad (3)$$

Lorsqu'on remplace la valeur de Cinb dans l'un ou l'autre membre de l'égalité (2) par la formule obtenue en (3) on obtient pour la dialysance D la formule suivante :

$$D = Qd\,(1 - \frac{C_{outd1} - C_{outd2}}{C_{ind1} - C_{ind2}}) \qquad (4)$$

Selon l'invention, le liquide circulant dans le compartiment 2 étant un liquide témoin de composition parfaitement connue, la valeur théorique $C_{inb}$ est connue.

Aussi, afin de vérifier le bon fonctionnement du capteur 23, l'unité de contrôle 30 impose, suivant un programme préétabli, une variation de la concentration du liquide de dialyse en commandant une variation de la vitesse de la pompe 17.

Dans un premier temps, le positionnement des vannes 24, 25, 26, 27, 28 et 29 est commandé de manière que le liquide de dialyse frais emprunte le circuit de dérivation 20 et baigne donc le capteur 23 avant de pénétrer dans l'hémodialyseur, alors que le liquide de dialyse usagé circule directement vers l'égout sans emprunter le circuit de dérivation 21. Le résultat de la mesure réalisée par le capteur 23 est transmis à l'unité 30 qui l'enregistre en tant que $C_{ind1}$. Lorsque la mesure est effectuée, l'unité 30 commande un changement de position des vannes 24, 25, 26, 27, 28 et 29 de manière que le liquide de dialyse frais suive la canalisation principale 12 pour pénétrer directement dans l'hémodialyseur et que le liquide de dialyse usagé emprunte le circuit de dérivation 21 pour baigner le capteur 23. Le résultat de cette mesure est alors transmis à l'unité 30 qui l'enregistre en tant que $C_{outd1}$.

L'unité 30 change alors la consigne de fonctionnement de la pompe 17 afin de changer la composition du liquide de dialyse et inverse le positionnement des vannes afin que la portion commune 22 soit à nouveau en communication avec la canalisation 12. La mesure effectuée par le capteur 23 est transmise à l'unité 30 qui l'enregistre en tant que $C_{ind2}$.

Puis l'unité 30 commande à nouveau l'inversion du positionnement des vannes afin que le capteur 23 puisse mesurer la concentration du liquide de dialyse usagé ; le résultat de la mesure est alors transmis et enregistré en tant que $C_{outd2}$.

A partir des résultats enregistrés, l'unité 30 calcule, au moyen de l'équation (3) une valeur pour la concentration du liquide circulant dans le compartiment 2.

Cette valeur calculée est comparée à la valeur théorique. Lorsque les deux valeurs coïncident, le bon fonctionnement du capteur 23 est confirmé. Dans le cas contraire, on peut prévoir le déclenchement d'une alarme ou l'émission d'un message avertissant l'opérateur du mauvais fonctionnement du capteur 23.

Lorsque le bon fonctionnement du capteur 23 a été ainsi confirmé, il est possible également de vérifier les performances de l'hémodialyseur en calculant grâce à la formule (4) la valeur de la dialysance obtenue à partir des valeurs $C_{ind1}$, $C_{ind2}$, $C_{outd1}$, $C_{outd2}$ transmises par le capteur 23 et la valeur du débit du liquide de dialyse indiquée par le débitmètre 15. Cette valeur calculée de la dialysance peut alors être comparée avec la valeur indiquée par le fabricant pour autant que les conditions d'ultrafiltration à travers la membrane soient sensiblement identiques.

La nature du capteur 23 peut varier ; il est en effet possible d'utiliser un conductimètre ainsi que n'importe quel capteur apte à mesurer la concentration d'une substance présente dans le liquide de dialyse. Dans le cas où les mesures sont effectuées par deux capteurs différents, un pour le liquide de dialyse frais et l'autre pour le liquide de dialyse usagé, la vérification est effectuée pour l'ensemble constitué par les 2 capteurs, mais ne permet pas, en cas de mauvais fonctionnement d'isoler le capteur défectueux.

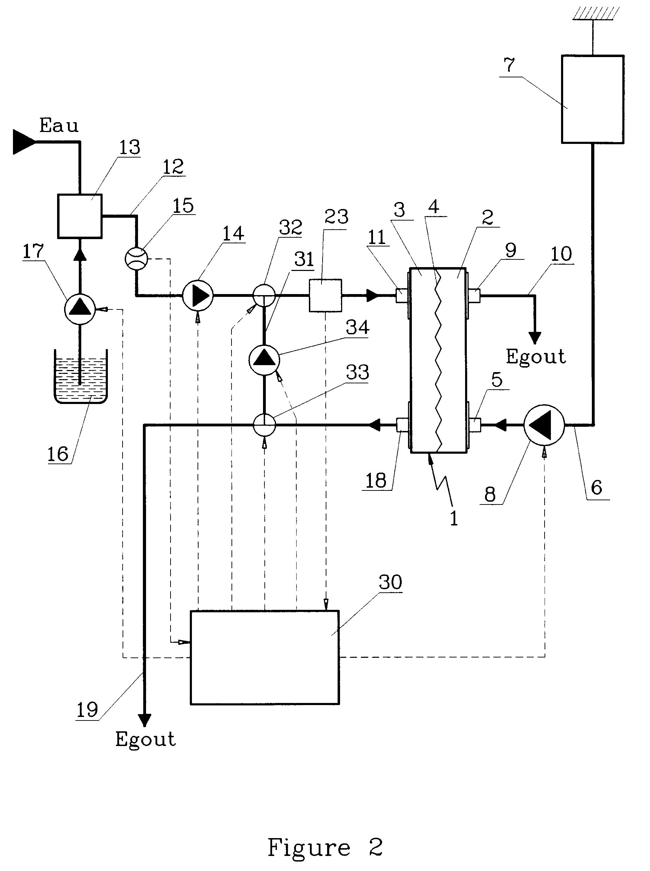

Selon le mode de réalisation illustré sur la figure 2, le circuit de liquide de dialyse est agencé de manière à réaliser une boucle de recirculation. A cette fin, une canalisation 31 munie d'une pompe 34 relie la canalisation 12 d'entrée du liquide de dialyse frais à la canalisation 19 d'évacuation du liquide de dialyse usagé. La mise en communication de la canalisation 31 avec la canalisation d'entrée 12 du liquide de dialyse frais est réalisée grâce à une vanne 3-voies 32 ; de même, la mise en communication de la canalisation 31 avec la canalisation d'évacuation 19 du liquide usagé est obtenue grâce à une vanne 3-voies 33. Dans cette configuration, le conductimètre 23 est situé sur la canalisation 12 en aval de la vanne 32. L'unité de contrôle 30 reçoit des informations du conductimètre 23 et contrôle le fonctionnement de la pompe 34 ainsi que le positionnement des vannes 32 et 33.

Le fonctionnement du rein artificiel ainsi décrit est le suivant. De même que dans le mode de réalisation précédent, le liquide témoin de conductivité connue, qui est présent dans le réservoir 7, est mis en circulation grâce à la pompe 8 commandée par l'unité 30. Simultanément, l'unité 30 commande le positionnement des vannes 32 et 33 de manière à permettre la circulation du liquide de dialyse directement de la source 13 à l'entrée 11 de l'hémodialyseur puis de la sortie 18 à l'égout. La pompe 34 ne fonctionne pas. La mise en oeuvre de la pompe 17 est commandée par l'unité 30 en fonction de la consigne de conductivité fixée préalablement par l'opérateur. L'unité 30 commande également le fonctionnement de la pompe 14 pour fournir un débit de liquide de 0,5 l/min, débit contrôlé par le débitmètre 15. Lorsque les canalisations 12 et 19 du circuit de liquide de dialyse sont remplies, l'unité 30 commande l'arrêt de la pompe 14, la mise en oeuvre de la pompe 34 ainsi qu'un changement de position des vannes 32 et 33 afin de mettre la canalisation 31 en communication avec la canalisation 12 et avec la canalisation 19 ; le liquide de dialyse recircule alors dans une boucle incluant le compartiment 3 de l'hémodialyseur au débit fixé par la pompe 34 qui est d'au moins 0,5 l/min. Les mesures effectuées par le conductimètre 23 sont transmises à l'unité 30. Tant que la valeur de conductivité varie, cela signifie que des échanges ont lieu par diffusion au travers de la membrane. Lorsque la conductivité mesurée par le conductimètre 23 ne change plus, cela signifie que le liquide de dialyse est en équilibre avec le liquide témoin circulant de l'autre côté de la membrane 4. La valeur alors mesurée est transmise à l'unité 30 qui l'enregistre en tant que valeur de conductivité à l'équilibre. L'unité 30 commande alors l'arrêt de la pompe 34, la remise en route de la pompe 14 et l'inversion de la position des vannes 32 et 33 afin d'isoler la canalisation 31 et permettre la circulation du liquide de dialyse directement de la source 13 à l'hémodialyseur 1 puis de l'hémodialyseur vers l'égout sans aucune recirculation.

L'unité 30 vérifie alors le bon fonctionnement du conductimètre en comparant la valeur enregistrée de conductivité du liquide de dialyse à l'équilibre avec la valeur connue de conductivité du liquide témoin.

De même que dans le mode de réalisation précédemment décrit, lorsque les deux valeurs coïncident, le bon fonctionnement du conductimètre 23 est confirmé alors que dans le cas contraire, on peut prévoir le déclenchement d'une alarme ou l'envoi d'un message d'avertissement à l'opérateur.

La présente invention n'est pas limitée aux exemples décrits. Différentes modifications peuvent y être apportées sans sortir de son cadre. Ainsi, il est possible notamment de prévoir que si la valeur calculée d'une caractéristique du liquide témoin ne correspond pas avec la valeur théorique, le capteur 23 soit recalibré de manière à faire coïncider les deux valeurs.

**Revendications**

1. Procédé de vérification du fonctionnement d'au moins un capteur (23) situé sur un circuit de liquide de dialyse d'un rein artificiel comportant un échangeur (1) à deux compartiments (2, 3) séparés par une membrane semi-perméable (4), un des compartiments (3) étant relié au circuit de liquide de dialyse (12,19) alors que l'autre compartiment (2) est destiné à être relié au circuit extracorporel (6,10) de sang, ce procédé consistant à :

   - faire circuler dans le circuit de liquide de dialyse (12, 19) un liquide de dialyse,
   - faire circuler dans le circuit extracorporel de sang un liquide témoin de caractéristiques connues,
   - effectuer au moins une mesure grâce à au moins un capteur (23) d'au moins une caractéristique du liquide de dialyse,
   - calculer à partir de cette mesure, la valeur théorique d'au moins une caractéristique du liquide témoin,
   - comparer la valeur théorique calculée à la valeur connue de la caractéristique du liquide témoin,
   - conclure au bon fonctionnement du capteur (23) au moyen duquel la mesure a été effectuée lorsque la valeur calculée de la caractéristique du liquide témoin est sensiblement égale à la valeur connue de la caractéristique du liquide témoin.

2. Procédé selon la revendication 1, caractérisé en ce qu'il consiste en outre à avertir l'opérateur lorsque la valeur théorique calculée n'est pas sensiblement égale à la valeur connue.

3. Procédé selon une des revendications précédentes caractérisé en ce qu'il consiste à faire recirculer le liquide de dialyse à l'intérieur de l'échangeur (1) jusqu'à ce qu'il soit en équilibre avec le liquide témoin et à effectuer la mesure d'au moins une caractéristique du liquide de dialyse lorsque l'équilibre est atteint.

4. Procédé selon une des revendications 1 ou 2 caractérisé en ce qu'il consiste en outre à faire varier la composition du liquide de dialyse et à mesurer pour chaque composition la valeur de la caractéristique en amont et en aval de l'échangeur (1).

5. Procédé selon une des revendications précédentes, caractérisé en ce que la caractéristique mesurée est la conductivité du liquide de dialyse.

6. Procédé selon une des revendications précédentes, caractérisé en ce que le liquide témoin est une solution stérile de sérum physiologique.

7. Procédé selon une des revendications précédentes, caractérisé en ce qu'il est mis en oeuvre lors de la phase de rinçage et d'amorçage de l'échangeur (1).

**Claims**

1. Method for checking the operation of at least one sensor (23) situated in a dialysis liquid circuit of an artificial kidney comprising an exchanger (1) with two compartments (2,3) separated by a semipermeable membrane (4), one of the compartments (3) being connected to the dialysis liquid circuit (12, 19) whilst the other compartment (2) is intended to be connected to the extracorporeal blood circuit (6, 10), this method lying in :

   - circulating a dialysis liquid in the dialysis liquid circuit (12, 19),
   - circulating a reference liquid of known characteristics in the extracorporeal blood circuit,
   - measuring at least one characteristic of the dialysis liquid by means of at least one sensor (23),
   - calculating the theoretical value of at least one characteristic of the reference liquid on the basis of the measurement,
   - comparing the calculated theoretical value with the known value of the characteristic of the reference liquid,
   - concluding that the sensor (23) with which the measurement has been taken is operating properly, when the calculated value of the characteristic of the reference liquid is substantially equal to the known value of the characteristic of the reference liquid.

2. Method according to claim 1, characterized in that it lies, moreover, in warning the operator when the calculated theoretical value is not substantially equal to the known value.

3. Method according to one of the preceding claims, characterized in that it lies in causing the dialysis liquid to recirculate inside the exchanger (1) until it is in equilibrium with the reference liquid, and in measuring at least one characteristic of the dialysis liquid when the equilibrium is reached.

4. Method according to one of claims 1 or 2, characterized in that it lies, moreover, in causing the composition of the dialysis liquid to vary and in measuring for each composition the value of the said characteristic upstream and downstream of the exchanger (1).

5. Method according to one of the preceding claims, characterized in that the measured characteristic is the conductivity of the dialysis liquid.

6. Method according to one of the preceding claims, characterized in that the reference liquid is a sterile physiological serum solution.

7. Method according to one of the preceding claims, characterized in that it is carried out at the time of the rinsing and priming stage of the exchanger (1).

**Patentansprüche**

1. Verfahren zur Funktionsprüfung von mindestens einem Sensor (23) an einem Dialysierflüssigkeitskreislauf einer künstlichen Niere mit einem Austauscher (1) mit zwei Kammern (2, 3), die durch eine semipermeable Membran (4) getrennt sind, wobei eine der Kammern (3) mit dem Dialysierflüssigkeitskreislauf (12, 19) verbunden ist, während die andere Kammer (2) mit dem extrakorporalen Blutkreislauf (6, 10) verbunden werden soll, wobei das Verfahren das Folgende umfaßt :

   - Umwälzen eine Dialysierflüssigkeit in dem Dialysierflüssigkeitskreislauf (12, 19),
   - Umwälzen eine Vergleichslösung mit bekannten Eigenschaften in dem extrakorporalen Blutkreislauf,
   - Durchführen mindestens eine Messung mindestens einer Eigenschaft der Dialysierflüssigkeit mittels mindestens eines Sensors (23) ,
   - Berechnen von dieser Messung ausgehend den Sollwert mindestens einer Eigenschaft der Vergleichslösung,
   - Vergleichen den berechneten Sollwert mit dem bekannten Wert der Eigenschaft der Vergleichslösung,
   - Schließen auf das ordnungsgemäße Funktionieren des Sensors (23), mittels dessen die Messung durchgeführt wurde, wenn der berechnete Wert der Eigenschaft der Vergleichslösung im wesentlichen gleich dem bekannten Wert der Eigenschaft der Vergleichslösung ist.

2. Verfahren nach Anspruch 1, dadurch gekennzeichnet, daß es darüber hinaus die Benachrichtigung der Bedienperson, wenn der berechnete Sollwert nicht im wesentlichen mit dem bekannten Wert übereinstimmt, umfaßt.

3. Verfahren nach einem der vorhergehenden Ansprüche, dadurch gekennzeichnet, daß es die Kreisführung der Dialysierflüssigkeit im Innern des Austauschers (1), bis sie sich mit der Vergleichslösung im Gleichgewicht befindet, und die Durchführung der Messung mindestens einer Eigenschaft der Dialysierflüssigkeit, wenn das Gleichgewicht erreicht ist, umfaßt.

4. Verfahren nach Anspruch 1 oder 2, dadurch gekennzeichnet, daß es darüber hinaus die Änderung der Zusammensetzung der Dialysierflüssigkeit und die Messung des Wertes der Eigenschaft für jede Zusammensetzung vor und hinter dem Austauscher (1) umfaßt.

5. Verfahren nach einem der vorhergehenden Ansprüche, dadurch gekennzeichnet, daß es sich bei der gemessenen Eigenschaft um die Leitfähigkeit der Dialysierflüssigkeit handelt.

6. Verfahren nach einem der vorhergehenden Ansprüche, dadurch gekennzeichnet, daß es sich bei der Vergleichslösung um eine sterile physiologische Kochsalzlösung handelt.

7. Verfahren nach einem der vorhergehenden Ansprüche, dadurch gekennzeichnet, daß es während der Spül- und Ansaugphase des Austauschers (1) durchgeführt wird.

Figure 1

Figure 2